# EUROPEAN PATENT SPECIFICATION

(11) **EP 1 666 134 B1**
(45) Date of publication and mention of the grant of the patent: **07.05.2008**
(21) Application number: 05025460.6
(22) Date of filing: 22.11.2005
(51) Int. Cl.: B01F 13/02, B01F 13/00, B01L 3/00

(54) **Hybridization chamber agitation device and method using pump and valves**
Rührvorrichtung und Verfahren einer Hybridisierungskammer umfassend Pump und Ventile
Dispositif d'agitation d'une chambre d'hybridation et procédé utilisant des pompes et des soupapes

(30) Priority: 06.12.2004 KR 2004101650
(43) Date of publication of application: 07.06.2006
(73) Proprietor: Samsung Electronics Co., Ltd., Suwon-si, Gyeonggi-Do (KR)
(72) Inventor: Jung, Sung-ouk, Yeongtong-gu Suwon-si Gyeonggi-do (KR); Huh, Nam, Seoul (KR); Lee, Soo-suk, Yeongtong-gu Suwon-si Gyeonggi-do (KR); Yoo, Chang-eun, Seoul (KR); Lee, Hun-joo, Jong-ro Seoul (KR)
(74) Representative: Grünecker, Kinkeldey, Stockmair & Schwanhäusser Anwaltssozietät

(56) References cited:
- WO-A-03/015923
- US-A1- 2003 013 184

## Description

### BACKGROUND OF THE INVENTION

### 1. Field of the Invention

The present invention relates to a hybridization chamber agitation device of a biochip, and more particularly, to an agitation device used to effectively agitate a solution in a hybridization chamber and a method of agitating using the same.

### 2. Description of the Related Art

A biochip is formed by affixing on a support a bimolecular probe to be analyzed with high density. The biomolecular probe may be DNA, protein, or the like. By detecting whether the probe is hybridized with a target material contained in a sample, genetic expression profile, genetic defects, protein distribution, reaction characteristics, or the like can be analyzed. Biochips are categorized into DNA chips, protein chips, and the like according to the type of probes used. In addition, biochips are categorized into micro-array chips affixed on solid supports and lab-on-a-chips affixed on micro-channels according to affixed subjects. Agitation and washing/drying systems are needed to attain effective hybridization between the target material contained in the sample, and the probe.

Conventional hybridization systems can be categorized into hybridization systems using pumps and hybridization systems using air and a solution. Examples of hybridization systems using pumps include a hybridization system using two membrane pumps disclosed in US Patent Publication No.2003/0013184 in the name of Tecan, and a hybridization system using a flow system pump disclosed in US Patent No. 6391623 in the name of Affymetrix. Examples of hybridization systems using air and a solution include a hybridization system using a rotary oven disclosed in EP Patent No.0933126 and a hybridization system using a rotary cartridge disclosed in US Patent Publication No. 2002/0001803.

In the conventional hybridization system using a flow system pump disclosed in US Patent No. 6391623 in the name of Affymetrix, illustrated in FIGS. 1A and 1B, a hybridization chamber is connected to a pump by a fluid delivery system, and hybridization is facilitated by the circulation of a fluid. In this case, however, the amount of the sample used must be large due to the use of a peristaltic pump and a circulation fluid channel. Because of this, after hybridization is performed for 16 hours using the hybstation, a used DNA chip must be washed and dried using a rotary oven.

In the conventional hybridization system using two membrane pumps disclosed in US Patent Publication No. 2003/0013184 filed by Tecan, illustrated in FIGS. 2A and 2B, two channels are connected to ends of the hybridization chamber. Each of the channels includes an agitation membrane and two micro pumps such that the target solution can be effectively hybridized with the probe on a chip. However, in order to obtain effective agitation in the chamber, the target solution is filled up to a chamber cover to mix the sample. Therefore, chamber is more contaminated.

In a similar manner, WO 03/015923 A discloses a system for mixing of fluids, comprising a hybridization chamber, wherein the fluids are moved by means of two mixing bladders which are deflected in an alternating direction with respect to the mixing chamber by means of a pressure source connected to the bladders via two air channels.

The conventional hybridization system disclosed in EP Patent No. 0933126, illustrated in FIG. 3, uses a rotary oven. In this case, hybridization is performed in the rotary oven for 16 hours. The period for hybridization may vary according to chip contents.

In the conventional hybridization system using a rotary cartridge disclosed in US Patent Publication No. 2002/0001803, as illustrated in FIGS. 4A and 4B, the hybridization is performed by rotating the cartridge. That is, after a chip cartridge is manufactured, a centrifugal force is used for hybridization.

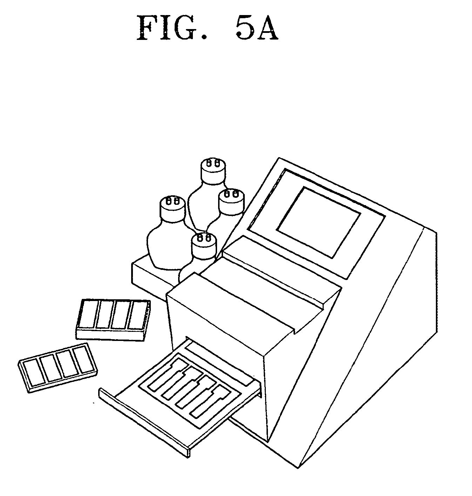

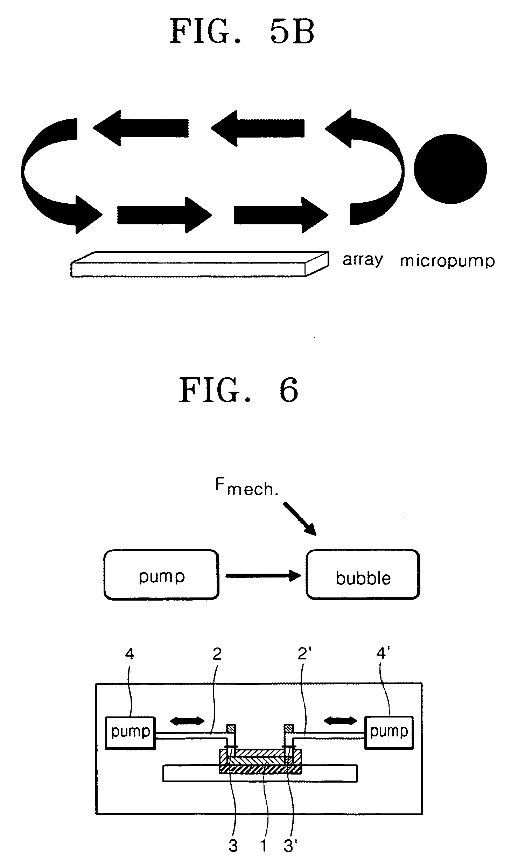

A conventional memorec A-hyb illustrated in FIG. 5 uses an active circulation by using a diaphragm pump. In this case, the amount of the sample used must be large, for example, about 220 µl, because the sample is circulated.

In order to solve these problems, the inventors of the present invention have confirmed that an agitation device can be effectively agitated by using a single pump and two valves disposed in an integrated channel and completed the present invention.

### SUMMARY OF THE INVENTION

The present invention provides an agitation device in accordance with claim 1 in which the amount of a sample required is decreased and a solution contained in a hybridization chamber is effectively hybridized.

The present invention also provides a method in accordance with claim 5 of effectively agitating a solution contained in the hybridization chamber using the agitation device.

According to an aspect of the present invention, there is provided an agitation device used to agitate a solution in a hybridization chamber, the agitation device including: the hybridization chamber; first and second air channels connected to ends of the hybridization chamber; a first valve disposed in the first air channel; a second valve disposed in the second air channel; an integrated air channel connecting the first and second air channels; and a pump disposed in the integrated air channel.

The hybridization chamber of the agitation device may be fixed in the agitation device, and is preferably a separable cartridge. A biochip, that is, a solid substrate on which probe molecules are fixed, may be inserted into the cartridge.

Any valve that can be opened and closed in response of electric signals can be used in the present invention. Preferably, a solenoid valve (series 075P obtained from bio-chemvalve Co.) can be used.

Any pump that can operate in response of electric signals can be used in the present invention. In particular, the pump may be a stepping motor-type micro pump.

According to another aspect of the present invention, there is provided a method of agitating a solution in a hybridization chamber using the above described agitation device, the method including: pumping away from a pump when the first valve is closed and the second valve is opened; pumping toward the pump when the first valve is opened and the second valve is closed; pumping away from the pump when the first valve is opened and the second valve is closed; and pumping toward the pump when the first valve is closed and the second valve is opened.

The agitation method according to the present invention and a conventional agitation method using the circulation of a solution in the hybridization chamber are different from each other in that in the present invention the solution contained in the hybridization chamber is not circulated and mixed by air in the air channel in a closed system. Therefore, the amount of the sample can be decreased to perform hybridization.

The break period between periods of pumping toward the pump may be in the range of 1 to 3 minutes. The presence of the break period results in an increase of hybridization efficiency and a constant hybridization chip intensity coefficient variation (CV).

The hybridization system may be constructed such that the solution contained in the hybridization chamber flows in a closed system by pumping away from and toward the micro pump and selectively closing the valves. Thus, the target solution can be effectively diffused onto the chip and bound to the probe affixed on the chip.

Although the size of the pump can be varied, the sizes of the valves and the pump may be a few to several tens of µms for ease of using the micro array and the lab-on-a-chip.

### BRIEF DESCRIPTION OF THE DRAWINGS

The above and other features and advantages of the present invention will become more apparent by describing in detail exemplary embodiments thereof with reference to the attached drawings in which:

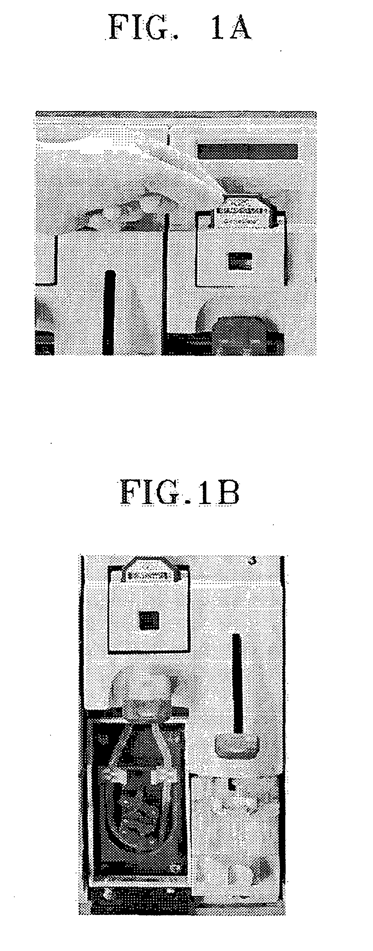

FIGS. 1A and 1B are views of a conventional hybridization device obtained from Affymetrix Co.;

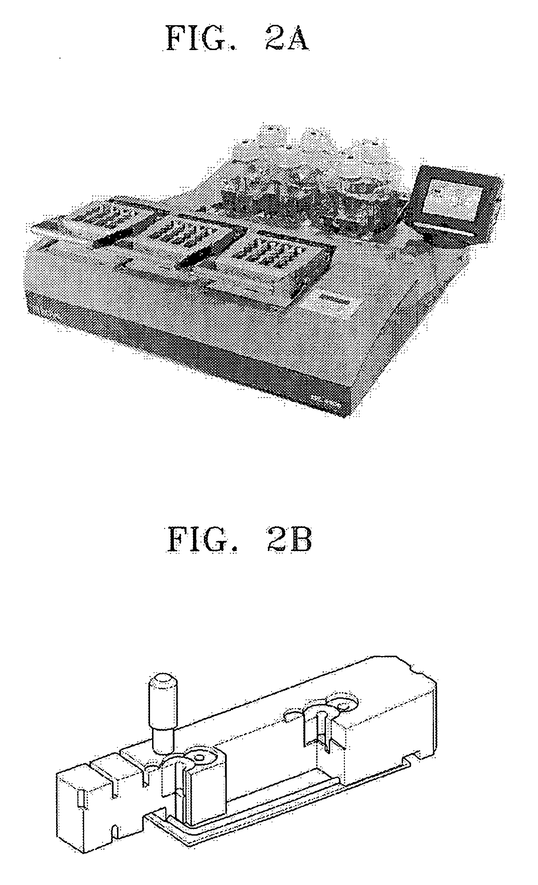

FIGS. 2A and 2B are views of a conventional hybridization device obtained from Tecan Co.;

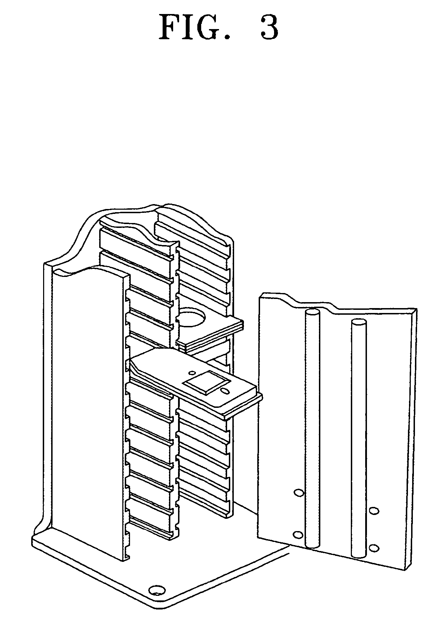

FIG. 3 is a view of a conventional hybridization device including a rotary oven;

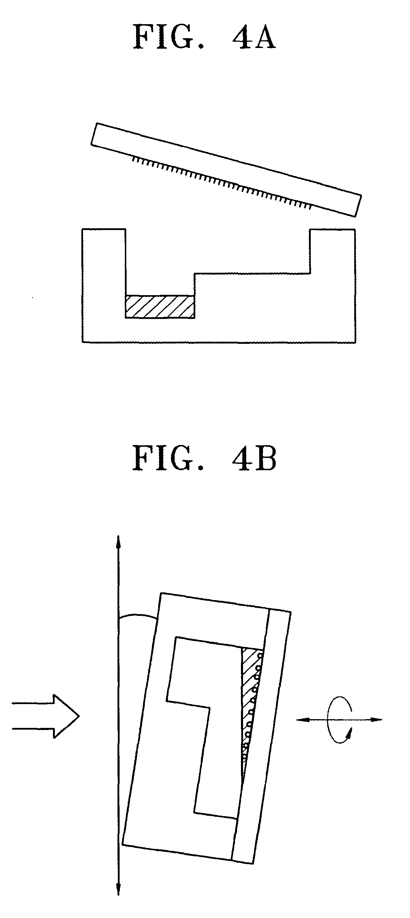

FIGS. 4A and 4B are views of a conventional hybridization device including a rotary cartridge;
FIGS. 5A and 5B are views of a conventional hybridization device using active circulation obtained from Memorec. Co;
FIG. 6 is a schematic view of a conventional hybridization device obtained from Tecan Co.;
FIG. 7 is a schematic view of an agitation device used to agitate a solution in a hybridization chamber according to an embodiment of the present invention;
FIG. 8 is a schematic diagram illustrating a method of agitating a hybridization chamber according to an embodiment of the present invention; and
FIG. 9 illustrates graphs of the intensity coefficient variation (CV) with respect to pushing time and break period of hybridization chips manufactured in Examples of the present invention.

### DETAILED DESCRIPTION OF THE INVENTION

Hereinafter, embodiments of the present invention will be described in detail with reference to the appended drawings.

FIG. 6 is a schematic view of a conventional hybridization device obtained from Tecan Co. Referring to FIG. 6, a hybridization chamber 1 has two ends respectively connected to channels 2 and 2'. The channels 2 and 2' are separated from the hybridization chamber 1 by membranes 3 and 3' such that contamination of the hybridization chamber 1 is prevented. An end of each of the channels 2 and 2' is connected to pumps 4 and 4'. A mechanical force applied to the pumps 4 and 4' pumps air inside the channels 2 and 2'. The pumped air agitates a solution contained in the hybridization chamber 1 through the membranes 3 and 3'. In this case, the membranes 3 and 3' must be included because the channels 2 and 2' can be contaminated due to the difficulty of precisely adjusting the force generated by the pumps 4 and 4'.

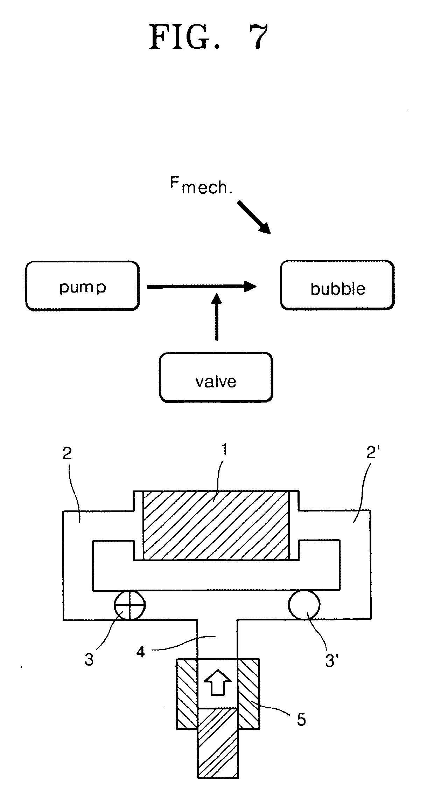

FIG. 7 is a schematic view of an agitation device used to agitate a solution in a hybridization chamber according to an embodiment of the present invention. Referring to FIG. 7, the agitation device according to an embodiment of the present invention includes a hybridization chamber 1, air channels 2 and 2' respectively connected to ends of the hybridization chamber 1, valves 3 and 3' respectively disposed in the air channels 2 and 2', an integrated air channel 4 connecting the air channels 2 and 2', and a pump 5 disposed in the integrated air channel 4. The hybridization device according to an embodiment of the present invention is different from the hybridization device obtained from Tecan Co. in that the hybridization device according to an embodiment of the present invention includes a 3-way valve that allows the flow of bubbles to be controlled by a single pump instead of two syringe pumps. Therefore, manufacturing costs are decreased, and contamination of the air channels 2 and 2' can be prevented as a result of the use of the single pump such that membranes are not needed.

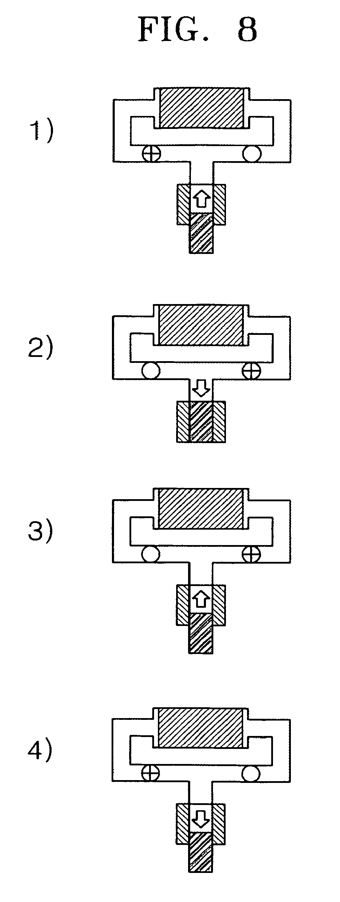

FIG. 8 is a schematic diagram illustrating a method of agitating a hybridization chamber according to an embodiment of the present invention. Referring to FIG. 8, the method includes: pumping air from a pump when one of the two valves is opened and the other valve is closed (illustrated in operation 1 of FIG. 8); pumping air to the pump when the opened valve and the closed valve are reversed (illustrated in operation 2 of FIG. 8); pumping air from the pump when no changes are made on the valves (illustrated in operation 3 of FIG. 8); and pumping air to the pump when the closed valve and the opened valve are again reversed (illustrated in operation 4 of FIG. 8). In FIG. 8, a closed valve is represented by a circle with '+' therein, an opened valve is represented by a circle, and the filled portion of the hybridization chamber is represented by the shaped section thereof. A solution contained in the hybridization chamber is agitated by repeating these operations.

Hereinafter, the present invention will be described in detail by explaining exemplary embodiments of the invention.

### Example 1: diffusion time during standing and agitation

An acryl structure into which a cartridge can be inserted was manufactured. A chamber was covered by a sealing pad and then the sealing pad was fixed by screws, forming the chamber with a height of 150 µm. The sealing pad was connected to two valves (series 075P obtained from bio-chemvalve Co.) and a pump (a stepping motor type pump obtained from uniflow Co.) via holes formed in the sealing pad, thus forming an agitation device according to an embodiment of the present invention illustrated in FIG. 7. 45µl of deionized (DI) water and 1 µl of ink were sequentially added to the completed structure via an inlet hole of the completed structure, and then the completed structure was connected to an agitation pump. Diffusion was measured by repeatedly alternating pumping directions with various pumping speeds.

A square chamber with a diameter of 17.3mm and a height of 150 µm was filled with Dl water, and then 5 µl of ink was added to the square chamber. Diffusion times were measured by repeatedly alternating pumping directions with various pumping speeds. A volume pumped from the pump was fixed at 4 µl and the period for which pumping direction is away from the pump was changed, thus changing the agitation rate.

Diffusion times and agitation rates are shown in Table 1.

**[Table 1]**

| Agitation rate | Time required |
|---|---|
| 0 ml/min | 13 hr |
| 0.12 ml/min | 25 min |
| 0.24 ml/min | 10 min |
| 2.4 ml/min | 5 min |

Referring to Table 1, it can be confirmed that the diffusion rate of the sample was heavily dependent on the agitation speed.

### Example 2: hybridization chip intensity coefficient variation (CV) according to period for pumping toward pump and break period

Hybridization chip intensity CV according to the period when pumping occurred away from the pump and break period was measured using the agitation device manufactured in Example 1. A chip was manufactured by cutting a silicon wafer coated with amine to a size matching the acryl structure manufactured in Example 1 and affixing identical probes (sequence: TGT TCT CTT GTC TTG) on the coated silicon wafer using a spotter. The resulting chip was affixed on the acryl structure. The chip was formed in a cartridge shape using an adhering agent such that the stability of the chip affixed on the acryl structure was increased (see Korean Patent Application No. 2004-0039981). Then, a target solution manufactured by mixing a probe (CAA GAC AAG AGA ACA) labeled with Cy3 into a hybridization buffer, was added to the hybridization chamber, and then agitated using the pump. When hybridization was completed, the cartridge was washed with a 3X SSPET buffer for 5 minutes and washed with a 1X SSPET buffer for 5 minutes. Then, an image of the chip was taken using a laser scanner, each spot in the image was quantified, and the intensity CV between spots was measured. The break period is a period between subsequent periods when air is pumped away from the pump.

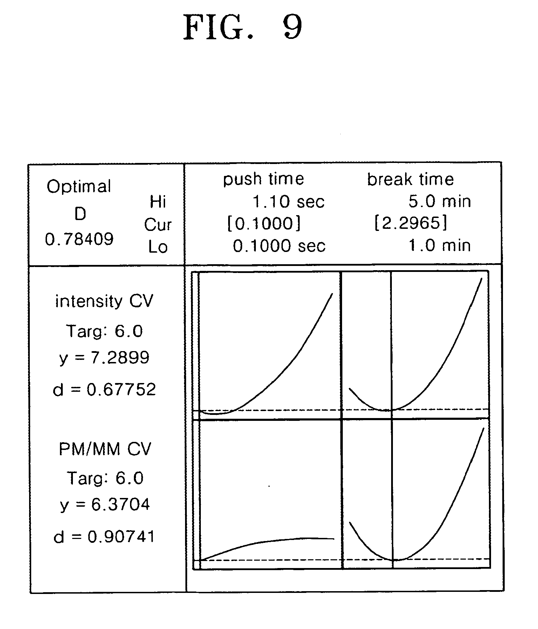

FIG. 9 illustrates graphs of the intensity CV of a hybridization chip with respect to the period when air was pumped away from the pump and the break period according to an embodiment of the present invention. CV is a coefficient variation and a lower CV is desirable. Referring to FIG. 9, when a volume of 4ul was pushed, the period when air was pumped was set to 0.1 sec, and the break period was set to 2.3 minutes, spot intensity CV and PM/MM ratio CV were minimized. The break period is needed and is preferably 1 to 3 minutes.

### Example 3: Use in Hybstation system

A MODY3 chip agitated using an agitation device according to an embodiment of the present invention was compared with that agitated using a conventional cover slide patch method. In this case, probes were used as indicated in Table 2.

**[Table 2]**

| WP | sequence | MP | Sequence |
|---|---|---|---|
| E02-01rwp | gacltgaccatc TTcgccacacg | E02-01rmp | gacttgaccateTCCgccacacg |
| E02-05rwp | tcccgctgtGGGatgttgggctgc | E02-05rmp | tcccgctgtGTGatottgtgctgc |
| E02-08rwp | tggtatcgaccACCtcccgctgt | E02-08rmp | tggtetcgaccATCtcccgctgt |
| E02-10rwp | ttgggaqgTGGgadggttgag | E02-10rmp | ttgggacaggTAGgactggttgag |

First, the probes, which correspond to a target hexane MODY3, were affixed on a substrate, thus forming a microarray. In detail, a wild probe (WP) and a mutant probe (MP) were added to a solution mixture of polyethyleneglycol (PEG), which has a molecular weight of 10,000, 0.025M (pH 10) of a sodium carbonate buffer, and formamide in a weight ratio of 1:1:2. The resulting solution was spotted on a silicon wafer using a bio-robot printer (Model No. PixSys 5500, obtained from Cartesian Technologies, Inc., CA, USA), and the silicon wafer was sit in a wet incubator at 37°C for 4 hours. Then, the surrounding noise was controlled such that portions of the silicon wafer that were not spotted were subjected to an appropriate reaction, thereby providing a negative charge to the amine group adhered to the surface of the wafer. Therefore, adherance of the target hexane to the silicon wafer could be prevented. The resulting silicon wafer was placed in a drying device. In the present example, the body or ends of the target hexane (tgggttctgccctttgcgctgggatggtgaagcttccagcc) was tagged with Cy3-dUTP as a fluorescent material. 187nM of the target hexane dissolved in 0.1 % of a 6xSSPET solvent (Saline Sodium Phosphate EDTA Buffer containing 0.1% of Trition X-100), and the microarray reacted at 37°C for 16 hours. Separately, 187nM of the target hexane dissolved in 0.1 % of a 6xSSPET solvent (Saline Sodium Phosphate EDTA Buffer containing 0.1% of Trition X-100), and the microarray were hybridized according to an embodiment of the present invention. Each of the chips was washed with 0.05 % 6xSSPET for 5 minutes and 0.05 % 3xSSPET for 5 minutes, dried at room temperature for 5 minutes, and then scanned by an Axon scanner (Model GenePix 4000B, Axon Instrument, Inc., CA, USA). The scanning data was analyzed using GenePix Pro 3.0 (obtained from Axon Instrument, Inc., CA, USA),

**[Table 3]**

| | Hyb time | Washing time | Drying time | Amount of sample used | Note |
|---|---|---|---|---|---|
| Traditional method | 4 hr | 10 min | less than 1 min | 60 µl | the other conditions are same |
| Hybstation method | 30 min | 6 min | 30 sec | 45 µl | |

Averages of spot intensities, PM/MM ratios, and coefficient variations (CVs) were measured by testing 20 copies of chips according to a conventional method and 20 copies of chips according to hybridization system. The results are shown in Table 4.

**[Table 4]**

| | Reference | | Hybstation method of present invention | |
|---|---|---|---|---|
| | Spot intensity | Ratio | Spot intensity | Ratio |
| Average | 18438.55 | 2.52 | 9852.38 | 2.31 |
| CV | 32.78 | 7.05 | 13.66 | 4.69 |

Referring to Table 4, intensity CV and PM/MM ratio CV were much smaller when an agitation device according to an embodiment of the present invention was used than when a conventional reference method was used.

As mentioned above, according to the present invention, a sample can be effectively diffused when performing hybridization using a DNA chip. Therefore, a probe can be effectively hybridized with a target. In addition, in agitation device according to the present invention, the amount of the sample to be agitated for hybridization is very small, for example, about 45ul. Therefore, the construction of the agitation device according to the present invention is inexpensive in comparison with conventional hybridization systems with two pumps. Further, in order to perform mixing in a closed system, only a pump and two valves are needed such that the agitation device according to the present invention can replace hybridization systems using a conventional Diaphragm pump or a conventional Peristaltic pump.

## Claims

1. An agitation device used to agitate a solution in a hybridization chamber, the agitation device comprising:
the hybridization chamber (1);
first (2) and second (2') air channels connected to ends of the hybridization chamber; and
a pump (5);
**characterized in that**
that a first valve (3) is disposed in the first air channel (2);
that a second valve (3') is disposed in the second air channel (2');
that an integrated air channel (4) connects the first (2) and second (2') air channels; and
that the pump (5) is disposed in the integrated air channel (4).

2. The agitation device of claim 1, wherein the hybridization chamber is a separable cartridge.

3. The agitation device of claim 1, wherein the valve is a solenoid valve.

4. The agitation device of claim 1, wherein the pump is a stepping motor-type micro pump.

5. A method of agitating a solution in a hybridization chamber using the agitation device of claim 1, the method comprising:
pumping away from a pump when the first valve is closed and the second valve is opened;
pumping toward the pump when the first valve is opened and the second valve is closed;
pumping away from the pump when the first valve is opened and the second valve is closed; and
pumping toward the pump when the first valve is closed and the second valve is opened.

6. The method of claim 5, wherein a solution contained in the hybridization chamber is agitated in a closed system without circulation through a channel.

7. The method of claim 5, wherein a break period between periods of pumping toward the pump is in the range of 1 to 3 minutes.

## Patentansprüche

1. Rührvorrichtung, die verwendet wird, um eine Lösung in einer Hybridisierungskammer zu rühren, wobei die Rührvorrichtung umfasst:
die Hybridisierungskammer (1);
einen ersten (2) und einen zweiten (2') Luftkanal, die mit Enden der Hybridisierungskammer verbunden sind; und
eine Pumpe (5);
**dadurch gekennzeichnet, dass**
ein erstes Ventil (3) in dem ersten Luftkanal (2) angeordnet ist;
ein zweites Ventil (3') in dem zweiten Luftkanal (2') angeordnet ist;
ein integrierter Luftkanal (4) den ersten (2) und zweiten (2') Luftkanal verbindet; und die Pumpe (5) in dem integrierten Luftkanal (4) angeordnet ist.

2. Rührvorrichtung nach Anspruch 1, wobei die Hybridisierungskammer eine abnehmbare Kartusche ist.

3. Rührvorrichtung nach Anspruch 1, wobei das Ventil ein Magnetventil ist.

4. Rührvorrichtung nach Anspruch 1, wobei die Pumpe eine Schrittmotor-Mikropumpe ist.

5. Verfahren zum Rühren einer Lösung in einer Hybridisierungskammer unter Verwendung der Rührvorrichtung nach Anspruch 1, wobei das Verfahren umfasst:
Pumpen von einer Pumpe weg, wenn das erste Ventil geschlossen ist und das zweite Ventil geöffnet ist;
Pumpen auf die Pumpe zu, wenn das erste Ventil geöffnet ist und das zweite Ventil geschlossen ist;
Pumpen von der Pumpe weg, wenn das erste Ventil geöffnet ist und das zweite Ventil geschlossen ist; und
Pumpen auf die Pumpe zu, wenn das erste Ventil geschlossen ist und das zweite Ventil geöffnet ist.

6. Verfahren nach Anspruch 5, wobei eine Lösung, die in der Hybridisierungskammer enthalten ist, in einem geschlossenen System ohne Zirkulation durch einen Kanal gerührt wird.

7. Verfahren nach Anspruch 5, wobei eine Unterbrechungsperiode zwischen Perioden des Pumpens auf die Pumpe zu im Bereich von 1 bis 3 Minuten liegt.

## Revendications

1. Dispositif d'agitation utilisé pour agiter une solution dans une chambre d'hybridation, le dispositif d'agitation comprenant :
la chambre d'hybridation (1) ;
des premier (2) et second (2') canaux d'air reliés aux extrémités de la chambre d'hybridation ; et
une pompe (5) ;
**caractérisé en ce que**
une première soupape (3) est disposée dans le premier canal d'air (2) ;
une seconde soupape (3') est disposée dans le second canal d'air (2') ;
un canal d'air intégré (4) relie les premier (2) et second (2') canaux d'air ; et
la pompe (5) est disposée dans le canal d'air intégré (4).

2. Dispositif d'agitation selon la revendication 1, dans lequel la chambre d'hybridation est une cartouche amovible.

3. Dispositif d'agitation selon la revendication 1, dans lequel la soupape est une électrovanne.

4. Dispositif d'agitation selon la revendication 1, dans lequel la pompe est une micropompe de type moteur pas à pas.

5. Procédé d'agitation d'une solution dans une chambre d'hybridation utilisant le dispositif d'agitation de la revendication 1, le procédé comprenant :
le pompage à l'écart d'une pompe lorsque la première soupape est fermée et la seconde soupape est ouverte ;
le pompage vers la pompe lorsque la première soupape est ouverte et la seconde soupape est fermée ;
le pompage à l'écart de la pompe lorsque la première soupape est ouverte et la seconde soupape est fermée ; et
le pompage vers la pompe lorsque la première soupape est fermée et la seconde soupape est ouverte.

6. Procédé selon la revendication 5, dans lequel une solution contenue dans la chambre d'hybridation est agitée dans un système fermé sans circulation à travers un canal.

7. Procédé selon la revendication 5, dans lequel une période de repos entre des périodes de pompage vers la pompe se situe dans la plage de 1 à 3 minutes.
